# EUROPEAN PATENT SPECIFICATION

(11) **EP 0 650 524 B1**
(45) Date of publication and mention of the grant of the patent: **25.06.2003**
(21) Application number: 94915098.1
(22) Date of filing: 22.04.1994
(51) Int. Cl.: C12Q 1/68, C12N 15/11

(54) **DETECTION OF THE CYTOLYSIN GENE WITH OLIGONUCLEOTIDES**
NACHWEIS DES CYTOLYSIN-GENES MIT OLIGONUKLEOTIDEN
DETECTION DU GENE DE CYTOLYSINE A L'AIDE DES OLIGONUCLEOTIDES

(30) Priority: 28.04.1993 US 54395; 30.04.1993 US 54480
(43) Date of publication of application: 03.05.1995
(73) Proprietor: MERCK PATENT GmbH, 64271 Darmstadt (DE)
(72) Inventor: GOEBEL, Werner Prof. Dr. Theodor-Boveri-Institut, D-97974 Würzburg (DE); HEFFRON, Fred, L., West Linn, OR 97068 (US); LIBBY, Stephen, J., San Diego, CA 92107 (US)
(86) International application number: EP9401262
(87) International publication number: WO94025595

(56) References cited:
- RES. MICROBIOL. vol. 141, no. 7-8 , 1990 pages 775 - 783 LIBBY, S.J. ET AL. 'Cloning and characterization of a cytotoxin gene from Salmonella typhimurium'
- EUR. J. BIOCHEM. vol. 192 , 1990 pages 609 - 620 WATANABE, K. ET AL. 'Primary structure of the oligo-1,6-glucosidase of Bacillus cereus ATCC7064 deduced from the nucleotide sequence of the cloned gene'
- VET. MICROBIOL. vol. 31 , 1992 pages 379 - 387 RAHMAN, H. ET AL. 'Salmonella cytotonic and cytolytic factors: their detection in Chinese hamster ovary cells and antigenic relatedness'
- PNAS vol. 91 , January 1994 , USA pages 489 - 493 LIBBY, S.J. ET AL. 'A cytolysin encoded by Salmonella is required for survival within macrophages' cited in the application

## Description

### Background of the Invention

Many Gram negative and Gram positive pathogenic microorganisms produce toxins or hemolysins that can lyse eukaryotic cells and contribute to their pathogenicity. S.A. Mims (1982), *The Pathogenesis of Infectious Disease* (Academic Press). The most intensively studied exotoxin produced by Gram negative pathogens are members of the RTX family and are reviewed in A. Ludwig and W. Goebel (1991), Genetic determinants of cytolytic toxins from gram-negative bacteria; in J.E. Alouf and J.H. Freev (eds.), Sourcebook of bacterial proteins, pp. 117-146, Academic Press, London; and in R.A. Welch (1991), *Mol. Micro.* **5**, 521-528, and V. Braun et al. (1991), *Critical Reviews in Microbiology* **18,** 115-158. The HlyA hemolysin present in strains of uropathogenic *E. Coli* is the best studied of these. HlyA increases *E. Coli* virulence in a rodent model of peritonitis. R.A. Welch et al. (1981), *Nature* **294,** 665-667. Although the precise mechanism by which this occurs is unclear, it is suggested that these toxins are involved in attenuating host phagocytic cell function. Welch et al., *supra*; S. Bhakdi et al. (1986), *Infect. Immun*. **52**, 63-69; S. Bhakdi et al. (1990), *J. Clin. Inv*. **85,** 1746-1753; S.J. Cavalleri et al. (1984), *Infect. Immun*. **37**, 966-974; and B. Konig et al. (1986), *Infect. Immun*. **54**, 886-892. Several facultative intracellular pathogens, including rickettsiae, shigellae, *Trypanosoma cruzi,* and *Listeria monocytogenes* share a common trait, i.e., to allow escape from the phagocytic vesicle of professional phagocytic cells. J.W. Moulder (1991), *Micro. Rev.* **55**, 143-190; J. Turco et al. (1991), *Infect. Immun.* **59**, 1647-1655; B.B. Finlay et al. (1989), *Micro. Rev.* **53,** 210-230; and N.W. Andrews et al. (1990), *Cell.* **61,** 1277-1287. A defined role in virulence is difficult to assign to most toxins, in part because of the lack of convenient animal models for many organisms. One exception is the listeriolysin made by *Listeria monocytogenes*. The toxin dissolves the phagocytic membrane, allowing the bacteria to escape into the cytoplasm; hemolysin negative mutants are avirulent. J.L. Gaillard et al. (1987), *Infect*. *Immun*. **55,** 1641-1646; S. Kathariou et al. (1987), *J. Bact*. **169**, 1291-1297; and D. Portnoy et al. (1989), *Infect. Immun*. **57**, 477-486. The infected cell remains intact, and the bacteria are protected from the immune defenses of the host. C.M. Hage-Chahine et al. (1992), *Infect. Immun*. **60**, 1415-1421. Transfer of the gene-encoding listeriolysin to the non-pathogenic bacterium *Bacillus subtilis* allows it to also escape the phagosome, suggesting that listeriolysin is both necessary and sufficient for this event. J. Bielecki et al. (1990), *Nature* **345**, 175-176.

It was observed that under the correct conditions, colonies of *Salmonella* were slightly hemolytic when cultured on blood agar plates. The segment of DNA encoding slyA was isolated from bacterial colonies expressing salmolysin by performing a series of deletion maps, carefully screening for hemolytic activity. The segment of DNA encoding the hemolytic activity was thus narrowed down to a specific region of the chromosome.
S.J. Libby et al. (1990), Res. Microbiol. **141**, 775-783, disclose a 1.6 kb EcoRV/Clal fragment subcloned from the Salmonella typhimurium ATCC14028. Expression of the fragment in E.coli yields a haemolytic protein.

### Summary of the Invention

The present invention relates to oligonucleotides selected from a DNA sequence according to SEQU.ID.NO.1 which is the nucleic acid sequence of the slyA gene, having a sequence according to SEQU.ID.NO.3, SEQU.ID.NO.4 or SEQU.ID.NO.5.

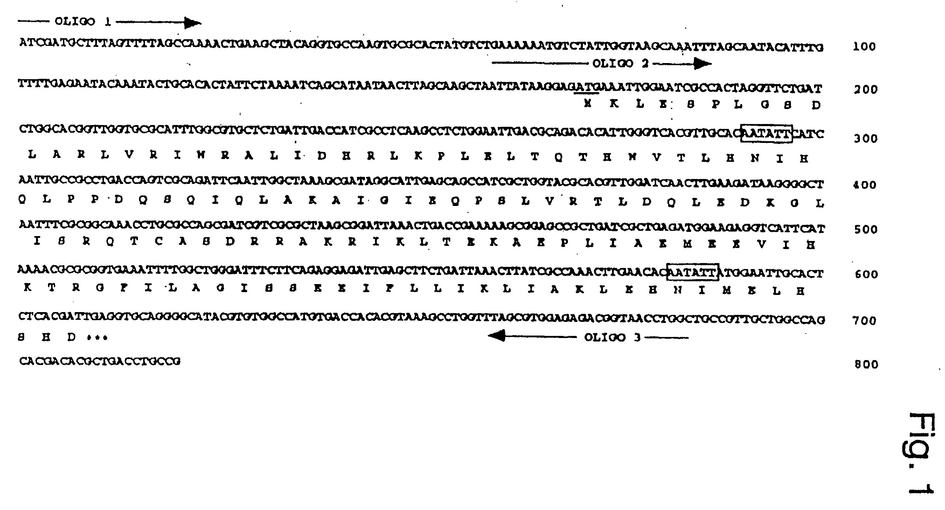

The present invention further relates to a method of detecting the presence of Salmonella comprising
- hybridizing an oligonucleotide according to claim 1 with a nucleic acid-containing sample suspected to contain Salmonella under conditions that allow selective detection of the slyA gene in that sample and
- determining the presence or absence of binding of the oligonucleotide, the presence of binding being indicative of the presence of Salmonella in the sample wherein said slyA gene is a nucleic acid having the sequence shown in Figure 1 or a sequence hybridizing to the DNA sequence of Figure 1 over its entire length and whereby the encoded protein has the biological activity of the protein encoded by the DNA sequence of Figure 1.

In a preferred embodiment of the method, the sample is a food stuff. In another preferred embodiment the sample is blood, serum, plasma, urine or feces.

The present invention further relates to a method of detecting the presence of Salmonella comprising
- hybridizing two different oligonucleotides comprising a DNA sequence which is specific for the DNA sequence of Figure 1, at least one of the oligonucleotides being an oligonucleotide according to claim 1 or their complements with a nucleic acid-containing sample suspected to contain Salmonella under conditions that allow selective detection of the slyA gene in that sample
- performing the polymerase chain reaction on the hybridized sample and
- determining the presence or absence of a DNA fragment formed by the polymerase chain reaction, the presence being indicative of the presence of Salmonella in the sample
wherein said slyA gene is a nucleic acid having the sequence shown in Figure 1 or a sequence hybridizing to the DNA sequence of Figure 1 over its entire length and whereby the encoded protein has the biological activity of the protein encoded by the DNA sequence of Figure 1.

The present invention further relates to a kit for detecting the presence of Salmonella comprising
- a first container having an oligonucleotide comprising a sequence according to claim 1 or a sequence which is complementary to a sequence of claim 1 and
- a second container having one or more components necessary for carrying out the reaction.

The present invention also relates to a kit for amplification of a region of DNA comprising
- a pair of single-stranded nucleic acid sense and antisense primers which are specific for the DNA sequence of Figure 1, at least one primer having a sequence or being complementary to a sequence according to claim 1
wherein said slyA gene is a nucleic acid having the sequences shown in Figure 1 or a sequence hybridizing to the DNA sequence of Figure 1 over its entire length and whereby the encoded protein has the biological activity of the protein encoded by the DNA sequence of Figure 1.

The terms "slyA gene" and "Salmolysin" as used herein refer to the gene depicted in Figure 1 [Sequ.lD.No. 1] and to the encoded protein. These terms refer as well to DNA sequences hybridizing to the nucleotide sequence of Figure 1 over its entire length and whereby the encoded protein products have the biological activity of the protein encoded by the DNA sequence of Figure 1. All variations of the slyA gene of Figure 1 and the encoded proteins, may be both naturally-occurring or man-made. The slyA genes may be found in *Salmonella*, but also in *Shigella, Escherichla*, and other bacterial strains.

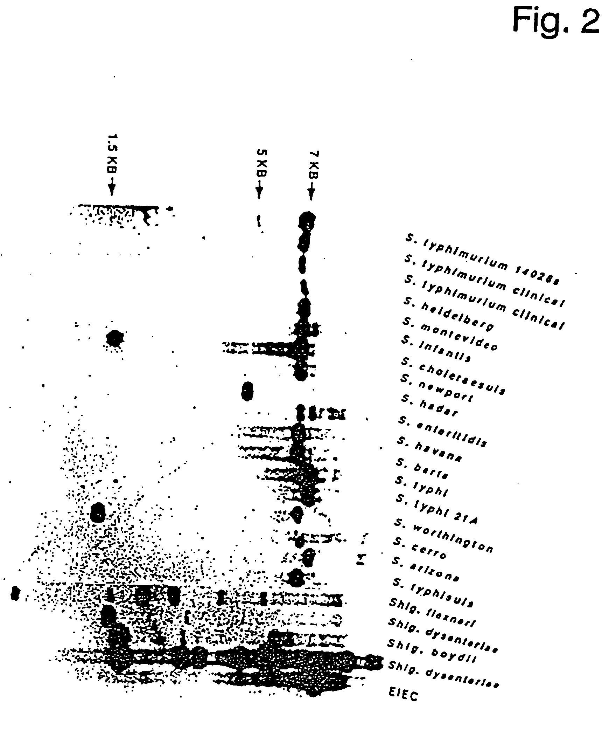

The slyA gene encodes a cytolysin, or salmolysin, with hemolytic and/or cytolytic activity.
These genes may be either naturally-occurring, chimeric, gene fragments thereof, or genetically-engineered, e.g., by site-directed mutagenesis, by chemical mutagens, radiation, transposons, or oligonucleotides. There may be DNA and/or RNA sequences which hybridize to the slyA gene or a derivative thereof (e.g., an oligonucleotide of the slyA gene or their complement sequences). Hybridizing sequences may be selected by their ability to hybridize specifically (e.g., selectively) the salmolysin gene or *a* fragment thereof under conditions which are generally suitable for selecting DNA sequences (e.g., see Molecular Cloning, Sambrook et al., 2nd Ed., 1989, especially 11.45-11.61). The source of DNA sequences capable of hybridizing with the slyA gene or gene fragment may be other bacterial strains, such as disclosed below in Figure 2, or, e.g., bacillus, aspergillus, amoeba, fungi, yeast, slime mold, insect, bird, mammals, and generally other living organisms and viruses. Although the *Salmonella* gene isolated herein is used as a representative example, it is recognized that other genes having cytolysin and/or hemolysin activity may be analogously isolated and used as described below.

The present invention relates to oligonucleotides of the slyA gene or a gene which hybridizes to the slyA gene. The oligonucleotides contain sequences from 5' and 3' non-coding regions of the slyA gene. The oligonucleotides may be employed, e.g., in the polymerase chain reaction, as probes, and in sequencing (e.g., see Molecular Cloning, Sambrook et al., 2nd Ed. 1989). The oligonucleotides may be used to amplify specific regions of the slyA gene by polymerase chain reaction for the purpose of identifying the presence of bacteria possessing the gene. For example, the presence in food stuffs, e.g. poultry, eggs, egg products, and meat, or in body fluids, e.g., blood, serum, saliva, feces, and urine, or in tissues, of strains having the slyA gene may be detected with a slyA probe by hybridization followed by the polymerase chain reaction. Since strains having the slyA gene may be pathogenic to living organisms, the ability to specifically and quickly detect their presence is especially advantageous for diagnostic and treatment purposes. For example, *Salmonella, Shigella* and EIEC can cause an inflammatory diarrhea. *Salmonella* more often causes a disseminated infection, while *Shigella* and EIEC remain within the colonic epithelium, rarely spreading past the lamina propria to produce a disseminated infection.

Oligonucleotide may be used as a hybridization or amplification probe as conventional in the art, including, in solution, on solid or semisolid supports, e.g., agarose (Northern or Southern) or polyacrylamide gels, nitrocellulose, nylon, and DEAE-cellulose membranes, microtiter wells, on tissue sections, whole tissue mounts, salivary and chromosome squashes, cells, or by PCR. The methods for hybridization may be performed, e.g., as in Molecular Cloning, Sambrook et al., 2nd Ed., 1989. For the purposes of performing PCR, oligonucleotides of a desired length may be selected which are specific for regions of the slyA gene for the purpose of amplifying and detecting the presence of a gene fragment in the sample DNA.

Thus, an aspect of the present invention is a method of detecting the presence of *Salmonella* having the slyA or a gene that cross-hybridizes with the slyA gene wherein a nucleic acid (Seq.I.D. No.3, 4, or 5) is hybridized with a sample containing nucleic acid in which the presence of the organism is to be detected and the presence or absence of binding is determined. The sample may be a food stuff, e.g. poultry, eggs, egg products, and meat, or blood, urine, feces or other body fluids and tissues which contain the organism having the slyA gene. The sample may also be prepared from the latter sources by standard methods of isolating or nucleic acid for test purposes. The method may involve hybridizing and detecting on solid support, e.g., gels and papers, or by PCR. Hybridization may be detected routinely, e.g., by radioactive or enzyme-conjugated or fluorophore-conjugated probes.

This invention is also a method of detecting the presence of the slyA gene in *Salmonella,* comprising hybridizing two different oligonucleotides comprising a sequence which is specific for the DNA sequence according to SEQU.ID.NO 1, at least one of the oligonucleotides having a sequence according to SEQU.ID.NO.3, SEQU.ID.NO.4 or SEQU.ID.NO.5 with a nucleic acid containing sample in which the presence of the slyA is to be detected, under conditions that allow selective detection of the slyA gene; performing the polymerase chain reaction on the hybridized sample; and detecting the presence or absence of a DNA fragment formed by the polymerase chain reaction. By "conditions that allow selective detection of the slyA gene" is meant that hybridization conditions are chosen so that the probe selectively detects a slyA gene in the nucleic acid sample. The selection of such conditions is routinely determined.

DNA encoding the salmolysin slyA gene may be obtained by chemical synthesis, by screening cDNA or genomic libraries prepared from mRNA or genomic DNA, respectively, from appropriate cells or cell line cultures, or by a combination of these procedures, using methods well known in the art. Screening of mRNA or genomic DNA may be carried out with oligonucleotide probes generated from the slyA gene sequence provided herein. Probes may be labeled with a detectable group such as a fluorescent group, a radionuclide, or a chemiluminescent group, or with an enzyme label in accordance with known procedures and used in conventional hybridization assays. Alternatively slyA gene sequences may be obtained by *in vitro* amplification, for example, the polymerase chain reaction (PCR), with oligonucleotide primers as provided herein (see US Patents 4,683,195 and 4,683,202 to Mullis).

5' sequences upstream and 3' sequences downstream from the slyA gene are also disclosed herein. For example, such sequences may be used as heterologous promoters or transcription terminators to control the expression of heterologous genes. Such uses may be accomplished conventionally, (e.g., see Methods in Enzymology, Volume 185, Gene Expression Technology, Ed. by D.V. Goeddel, 1990), but substituting the novel sequences of the slyA gene.

The present invention is also a kit comprising oligonucleotides which are specific for the salmolysin gene, i.e. oligo 1 [Sequ.ID.No. 3], oligo 2 [Sequ.ID.No. 4], or oligo 3 [Sequ.ID.No. 5], according to Figure 1, or their complement thereof. The kit may be used to detect the presence of *Salmonella* expressing a slyA gene (for diagnostic purposes.) The kit may include oligonucleotides, buffers, and other reagents used to detect the presence of a nucleic sequence which hybridizes to the oligonucleotides. The oligonucleotides may contain conjugated fluorophores or enzymes, in which case the kit would include the reagents necessary to detect the fluorophores or enzymes. The kit may also be used in conjunction with a polymerase chain reaction, comprising the reagents to carry out the reaction, including, e.g., oligonucleotide primers to a slyA gene, buffer, DNA polymerase and dNTPs. (See Sambrook et al. supra.)
A. **Strains and Media:** Wild-type ATCC strain *Salmonella typhimurium* 14028s was used in all virulence studies. Clinical isolates of *Salmonella* were obtained from the State of California Health Laboratory or the County of San Diego Health Laboratory. Bacteria were cultivated in Luria Bertani medium. Blood agar plates were made in trypticase soy agar (TSA Difco) containing 4% defibrinated sheep red blood cells (Colorado Serum, Denver). Clinical isolates obtained for homology studies included: *Yersinia* sp., *Legionella, Chlamydia*, *Pasteurella*, *Acinetobacter, Haemophilus*, *Proteus, Klebsiella, Neisseria* sp., *Citrobacter, Campylobacter*, *Franciscella, Brucella, Listeria, Serupia* sp., *E. Coli* K-12, and *Aeromonas*.
B. **Molecular Techniques:** A Sau3A partial digest cosmid library of *Salmonella typhimurium* 14028s was constructed in the vector pLARF2 [A.M. Friedman et al. (1982), *Gene.* **18,** 289-296] and packaged into lambda particles (Stratagene, La Jolla, California). *E. Coli* LE392 was used as the host for the cosmid library and was plated onto TSA blood plates containing 20 µg/ml tetracycline and incubated for 36 hours at 37°C to detect zones of hemolysis. A strongly hemolytic colony was chosen for further characterization. Subcloning, sequencing, and Southern analysis were performed by standard techniques [J. Sambrook et al. (1989), *Molecular Cloning: A Laboratory Manual*, Second Edition (Cold Spring Harbor: Cold Spring Laboratory Press)] in either M13 mp19 or pKS (Stratagene, LaJolla, California) using the Sequenase kit (United States Biochemicals). Sequence manipulation and data base searches were performed using IBI MacVector and GenBank.
C. **Chromosomal Location of the *sly* Gene and Southern Hybridizations:** A series of "locked-in" Mud-P22 phage insertions at 3-minute overlapping intervals around the *Salmonella typhimurium* chromosome has been created. N.R. Benson et al. (1992), *J. Bact*. **175**, 1673-1681, and P. Youderian et al. (1988), *Genetics* **118,** 581-592. Each strain packages up to 120 kb (about 3 minutes) of adjacent chromosome when induced with mitomycin C at 1 µg/ml. Phage particles were pelleted from the resulting lysates, and DNA was prepared by phenol extraction. One µ g of DNA from each region of the chromosome was immobilized on Nytran using a slot blot apparatus (Schleicher and Schuell) and hybridized with the salmolysin structural PCR amplified gene. All Southern hybridizations were done in 6x SSPE, 10x Denhardts, 0.5% SDS at 65°C and filters washed in 0.1x SSPE at 65°C. Hybridization, PCR, and other general methods and solutions which are useful in the present invention are described in Sambrook et al., *supra.*
D. **Disruption of the *sly* Gene:** The *sly* gene was disrupted by homologous recombination insertion of a suicide vector derived from the RK2 replicon, pFR10. R.C. Roberts et al. (1990), *J. Bact.* **172,** 6204-6216. The essential replication gene, *trf*A, was deleted from this vector and replaced with an internal *Ssp1* fragment of *sly*A. The suicide vector-*sly* fragment, pSL2145, was maintained in *E. Coli* S17-1 [R. Simon et al. (1983), *Molecular Genetics of Plato-Microbe I Interactions,* A. Puhlar, ed. (St. Paul Minnesota: APS Press), pp. 98-106] and transferred to wild-type *Salmonella* by conjugation. Transconjugates were selected on Brilliant green agar containing sulfadiazine (80 µg/ml) and penicillin (300 µg/ml). Because *Salmonella* recipient cells lack *tri*A, penicillin resistance can only be stably maintained in recipients by homologous recombination between the internal *sly* fragment on the suicide plasmid and the *sly* gene on the chromosome, resulting in *sly* disruption. Chromosomal DNA was prepared from several penicillin-resistant colonies, restricted with *Pst*I, *EcoR*V, Clal, and *EcoR*V*ICla*I, transferred to a membrane, and probed with the ³²P-labelled PCR amplified *sly*A gene to confirm the interruption of *sly*A. PCR may be performed as described in Innis et al. (1990), *PCR Protocol: A Guide to Methods and Applications* (Academic Press). All penicillin-resistance colonies examined showed disruption of the *sly*A gene.
E. **Virulence Determination of *sly* Mutants:** Overnight cultures of 14028s (wild type) and SL2161 (*sly*A-) were grown in LB and LB containing penicillin (200 µg/ml), respectively, and given orally to mice in 200 µl doses with a feeding cannula. Intraperitoneal and intravenous inoculations were given with bacteria washed and diluted in phosphate-buffered saline. LD₅₀ values were determined over a four-week period. Course of infection studies were performed by homogenizing tissues in a Stomacher (Tekmar) from infected mice in sterile water, diluting the homogenates in phosphate-buffered saline, and plating or LB agar to determine the number of bacteria per organ.
F. **Expression and Partial Purification of a Salmolysin:** Methods for the expression of cloned genes in bacteria are well known. To obtain high level expression of a cloned gene in a prokaryotic system, it is often essential to construct expression vectors which contain, at the minimum, a strong promoter to direct mRNA transcription termination. Examples of regulatory regions suitable for this purpose are the promoter and operator region of the *E. coli* β-galactosidase gene, the *E. coli* tryptophan biosynthetic pathway, a T7 or other RNA polymerase promoter or the leftward promoter from the phage lambda. The inclusion of selection markers in DNA vectors transformed in *E. coli* are useful. Examples of such markers include the genes specifying resistance to ampicillin, tetracycline, or chloramphenicol. See Sambrook, supra, for details concerning selection markers and promoters for use in *E. coli*.
   The oligonucleotide primers shown in Figure 1 [Sequ.ID.No's. 3, 4, and 5] were used to amplify a 515 bp fragment that contained an *Nde*l site modified at the start codon and cloned into pRK172 [A.H. Rosenberg et al. (1987), *Gene.* **56**, 125-136] at the *Nde*l site, placing salmolysin expression under the control of the T7 RNA polymerase promoter. The construct, pSL2042, was transformed into *E. Coli* B121 DE3 [F.W. Studier et al. (1990), *Methods in Enzymology* (Academic Press, San Diego), Vol. 185, pp. 6-89] and expression of salmolysin was induced by 1 mM IPTG (isopropylbetagalactoside) in the presence of 200 µg/ml rifamplcin and 50 µCl/ml ³⁵S-methionine. Proteins were separated by 12% SDS-PAGE, gel stained with Coomassie Brilliant Blue, dried, and exposed to X-ray film. Salmolysin was released from *E. Coli* containing pSL 1117 by the osmotic shock procedure of Neu and Heppel. H.C. Neu et al. (1965), *J. Biol. Chem.* **240,** 3685-3692. Proteins were separated by FPLC monoQ anion exchange (Pharmacia) with a 0-1 M NaCl gradient, 50 mM Tris-HCI, pH 7.5 and 2 µg/ml of PMSF. Column fractions were assayed for hemolytic activity by adding 50 µl of each fraction to 700 µl of 10% defibrinated, washed sheep erythrocytes and incubated at 37°C for 1 hour. The amount of released hemoglobin was determined by measuring the optical density of the supernatant at 595 nm. Salmolysin eluted at 150 mM NaCI. Salmolysin could be further purified using preparative isoelectric focusing with a Fletofor (BioRad): all hemolytic activity focused at a pl of 5.5. A hemolytic unit of salmolysin was defined as the amount of partially purified salmolysin required to lyse 50% of a suspension of 10% erythrocytes at 37°C in 1 hour. One hemolytic unit of salmolysin was heated to 65°C for 15 minutes, then assayed for activity. EDTA (50 mM final) was added to one hemolytic unit of salmolysin and treated as above. Phospholipase D and cholesterol oxidase activities were assayed by the method described in J.G. Songer et al. (1990), *Infect. Immun*. **58,** 131-136.
G. **Macrophage Preparation and Infection:** Peritoneal macrophages were elicited from Balb/c mice by intraperitoneal injection of proteous peptone, cultured, and infected with wild type and the salmolysin mutant as described in N.A. Buchmeier et al. (1989), *Infect. Immun*. **57**, 1-7.
H. **General Methods of Molecular Cloning and Genetic Engineering:** This invention embraces molecular genetic manipulations that can be achieved in a variety of known ways. The recombinant cells, plasmids, and DNA sequences of the present invention provide a means to produce pharmaceutically useful compounds wherein the compound is a salmolysin.
   Generally, the definitions of nomenclature and descriptions of general laboratory procedures used in this application can be found in J. Sambrook et al., Molecular Cloning, A Laboratory Manual, (1989) Cold Spring Harbor Laboratory, Cold Spring Harbor, New York. The manual is hereinafter referred to as Sambrook and is hereby incorporated by reference. In addition, Ausubel et al., eds., Current Protocols in Molecular Biology, (1987 and periodic updates), Greene Publishing Associates, Wiley-lnterscience, New York, discloses methods useful in the present application.
   All enzymes are used according to the manufacturer's instructions.
   Oligonucleotides that are not commercially available can be chemically synthesized according to the solid phase phosphoramidite triester method first described by S.L. Beaucage and M.H. Caruthers, (1981) Tetrahedron Letts., 22(20):1859-1862 using an automated synthesizer, as described in D.R. Needham-VanDevanter et al., (1984) Nucleic Acids Res., 12:6159-6168. Purification of oligonucleotides is by either native acrylamide gel electrophoresis or by anion-exchange HPLC as described in Pearson, J.D., and Regnier, F.E. (1983) J. Chrom., 255:137-149. Nucleotide sizes are given in either kilobases (kb) or base pairs (bp). These are estimates derived from agarose or acrylamide gel electrophoresis or from published DNA sequences.
   The sequence of the cloned genes and synthetic oligonucleotides can be verified using the chemical degradation method of Maxam, A.M., et al., (1980) Methods in Enzymology, 65:499-560, or similar methods. The sequence can be confirmed after the assembly of the oligonucleotide fragments into the double-stranded DNA sequence using the method of Maxam and Gilbert, supra, or the chain termination method for sequencing double-stranded templates of Wallace, R.B., et al., (1981) Gene, 16:21-26. Southern Blot hybridization techniques were carried out according to Southern et al., (1975) J. Mol. Biol., 98:503.
   Novel peptides and genes may be created by *in vitro* mutagenesis. Target genes are isolated in intermediate vectors and cloned for amplification in prokaryotes such as *E. coli*, *Bacillus,* or *Streptomyces.* Most preferred is *E. coli* because that organism is easy to culture and more fully understood than other species of prokaryotes. The Sambrook manual contains methodology, e.g., to conduct cloning and genetic engineering methods, *E*. *coli* strains may be grown in Luria broth (LB) with glucose, or M9 medium supplemented with glucose and acid-hydrolyzed casein amino acids. Strains with resistance to antibiotics were maintained at the drug concentrations described in Sambrook. Transformations may be performed according to the method described by Morrison, D.A. (1977) J. Bact., 132:349-351 or by Clark-Curtiss, J.E., and Curtiss, R. (1983) Methods in Enzymology, 101:347-362, Eds. R. Wu et al., Academic Press, New York.
I. **Peptides and Antibodies.** The peptides can be prepared by conventional processes for synthesizing peptides; more specifically, using processes as described in Schroder and Lubke, The Peptides, Vol. 1, published by Academic Press, New York (1966) or Izumiya, et al., Synthesis of Peptides, published by Maruzen Publishing Co., Ltd., (1975). For example, an azide process, an acid chloride process, a symmetric anhydride process, a mixed anhydride process, a DCC process, an active ester process (for example: p-nitrophenyl ester, N-hydroxysuccinimide ester, or cyanomethyl ester), a carbodiimidazole process, an oxidative-reductive process, or a DCC/additive process can be used. Solid phase and solution phase syntheses are both applicable to the foregoing processes.

Typically peptide synthesis is generally performed either by a so-called stepwise process which comprises condensing an amino acid to the terminal amino acid, one by one in sequence, or by coupling peptide fragments to the terminal amino acid. Amino groups that are not being used in the coupling reaction and other reactive groups must be protected to prevent coupling at an incorrect location. Procedures of protecting such groups and of removing the protective groups are known in the art.

In case that a solid phase synthesis is adopted, the C-terminal amino acid is bound to an insoluble carrier or support through its carboxyl group. The insoluble carrier is not particularly limited as long as it has a binding capability to a reactive carboxyl group. Examples of such insoluble carriers include halomethyl resins of polystyrene, such as chloromethyl resins, phenol resins, tert-alkyloxycarbonyl-hydrazidated resins, and the like.

In general, antibodies may be obtained by injecting the desired immunogen or antigen into a wide variety of vertebrates, in accordance with conventional techniques. Suitable vertebrates include mice, rats, rabbits, sheep, and goats, with mice being preferred. Usually, the animals are bled periodically with the successive bleeds having improved titer and specificity. The antigens may be injected intramuscularly, intraperitoneally, subcutaneously, or the like. Chimeric antibodies (mouse human hybrids) made by genetic engineering are also contemplated by this invention.

Polyctonal antibodies are prepared by hyper-immunization of the animal with antigen. Then the blood of the animal is collected shortly after the repeated immunizations and the gamma globulin is isolated. Suitable methods for preparing polyclonal antibodies are described in the Handbook of Experimental Immunology, 3d edition, (ed. Weir, 1978).

To obtain monoclonal antibodies, spleen cells from the immunized vertebrate demonstrating the desired antibody response are immortalized. The manner of immortalization is not critical, but the most common method is fusion with a myeloma fusion partner. Other techniques of immortalization include EBV transformation, transformation with bare DNA, such as oncogenes or retroviruses, or any other method which provides for stable maintenance of the cell line and production of monoclonal antibodies. The general process for obtaining monoclonal antibodies is described by Kohler and Milstein, in Nature, 256, 495-497 (1975), which is herein incorporated by reference. Human monoclonal antibodies may be obtained by fusion of the spleen cells with an appropriate human fusion partner, such as WI-L2, described in European Application No. 82.301103.6, the relevant portions of which are hereby incorporated by reference. A detailed technique for producing mouse x mouse monoclonal antibodies is taught by Oi and Herzenberg, in Selected Methods in Cellular Immunology, 351-372 (eds. Mishell and Shiigi, 1980), which also is herein incorporated by reference. The resulting hybridomas are screened to isolate individual clones, each of which secretes a single antibody species capable of recognizing the antigen.

The peptides and/or antibodies may be used without modification or may be modified in a variety of ways, for example, by labeling. Labeling is intended to mean joining, either covalently or non-covalently, a moiety which directly or indirectly provides for a means of detection. A wide variety of labels are known and include: radionuclides, enzymes, substrates, cofactors, inhibitors, fluorescers, chemiluminescers, magnetic particles and the like.

Many of the techniques for linking the peptides to suitable labels involve the use of their carboxyl groups, through the use of carbodiimide or active esters to form peptide bonds; the formation of sulfides by reaction of a mercapto group with an activated halogen, such as chloromethylstyrene or activated olefin, such as maleimide; formation of a secondary amine by reaction of an amino group with a dialdehyde such as glutaraldehyde, or the like, followed by reduction with sodium cyanoborohydride, or the like.

An amino group-protected amino acid is bound in sequence through condensation of its reactive carboxyl group to the terminal amino group of the growing chain. After synthesizing the complete sequence, the peptide is removed from the insoluble carrier to yield free the peptide. This solid-phase approach is generally described by Merrifield, et al. in J. Am. Chem. Soc., 85, 2149-2156 (1963).

Peptides can also be prepared through DNA techniques. The amino acid sequence of the desired peptide is used to deduce the codon sequence for the single-stranded DNA, synthesized using conventional synthetic techniques (including multiple gene copy techniques), then the double-stranded DNA is prepared and inserted at a suitable site in a cloning vehicle, vector, or plasmid. An appropriate organism, such as bacteria cells, yeast cells, or mammalian cells, is transformed to obtain expression of the desired peptide.

The prepared peptides can be isolated and purified from the reaction mixture by means of peptide separation, for example, by extraction, counter-current distribution, column chromatography, high performance liquid chromatography, and the like.

### Brief Description of the Drawings

The present invention will be better understood when considered in conjunction with the accompanying drawings, in which like reference characters designate the same or similar parts throughout the several views.
**Figure 1. Salmolysin Sequence.** The sequence of the *sly* gene. The initiation codon is underlined and the termination codon is denoted by *. Sequences surrounded by the two boxes are *Sspl* sites used to construct pSL2145. The DNA sequence is given as [Sequ.ID.No. 1] and the corresponding protein sequence of Salmolysin as [Sequ.ID.No. 2]. Primers used for PCR reactions are shown (Oligo 1-3; [Sequ.ID.No's. 3-5]).
**Figure 2. Conservation of the *sly* Gene.** Chromosomal DNA from clinical isolates of *Salmonella* was isolated, restricted with *Pstl* transferred to a Nytran, and probed with the labeled PCR amplified *sly*A fragment. A single 7 kb fragment hybridized to the probe with the exception of *S. worthington, S. infantis,* and *S. enteritidis*. The *sly*A gene may be multicopy in these serotypes. A 5 kb fragment hybridizes with *S. hadar.* These data are summarized in Table 1.

In the foregoing and in the following examples, all temperatures are set forth uncorrected in degrees Celsius and unless otherwise indicated, all parts and percentages are by weight.

### EXAMPLES

### Example 1: CLONING THE SALMOLYSIN GENE

Although *Salmonella* is not usually hemolytic on laboratory culture media, weak zones of hemolysis around some colonies, especially in clinical isolates were occasionally observed. It was suspected that this variable phenomenon might be highly regulated and the putative hemolysin might only be expressed *in vivo.* Therefore, it was attempted to clone the cognate gene away from its normal regulatory elements. A cosmid clone bank was made from *S*. *typhimurium* ATCC 14028 in pLAFR2, packaged into bacteriophage lambda *in vitro*, introduced by infection into *E. coli* strain LE392, and screened for hemolytic activity on blood agar plates. A detailed restriction map of a representative hemolytic clone was prepared and a series of deletions constructed to identify the location of the gene encoding the hemolysin. It was found that a 1.4 kb *Clall*EcoRV fragment (pSL1117) of the original 25 kb cosmid clone was required for hemolotic activity in *E*. *coli*. The nucleotide sequence of the 1.4 kb fragment was determined and analysis of the sequence adjacent to the *Cial* site revealed a 436 base open reading frame that could encode a protein of 16,747 daltons. The polymerase chain reaction (PCR), see, e.g., Innis et al., (1990), was used to amplify a 680 base pair region from chromosomal DNA encompassing the open reading frame. The sequence of this region is shown in Figure 1 [Sequ.ID.No. 1]. The PCR derived fragment was cloned into pKS (Stratagene, La Jolla, CA) and screened for hemolytic activity. This clone, pSL2070, was equally hemolytic as pSL1117, indicating that the 680 base-pair fragment contained all the genetic information necessary for the hemolytic phenotype in *E. coli*. The deduced protein sequence was used to search a translated version of the Genbank DNA database (updated August 1992). The closest matches were compared with *sly*A using the Fustell protein comparison matrix but no significant short homologies were found. Surprisingly, analysis of the salmolysin coding sequence did not reveal a classical signal sequence. However, it is still possible that salmolysin is exported to the outer membrane or extracellularly *in vivo* by a sec-independent pathway similar to export of the HlyA [R. A. Welch, Mol. Micro. Vol. 5, pp. 521-528 (1991) and Braun, V. & T. Focareta, Critical Reviews in Microbiology, Vol. 18, pp. 115-158 (1991)]. Thus, a single gene, which we call *sly*A for salmolysin, is sufficient to confer hemolytic activity on laboratory strains of *E. coli*.

### EXAMPLE 2. MAPPING AND CONSERVATION OF THE SALMOLYSIN GENE IN SALMONELLA sp.

Since the map location for many *Salmonella* genes have been determined, it was decided to map *sly*A to determine whether it corresponds to a genetically mapped but un-sequenced gene. The precise genetic location of the *sly*A gene was determined using a modification of the procedure described in [N.R. Benson and B.S. Goldman, J. Bact., Vol. 175, pp. 1673-1681 (1992) and P. Youdenan et al. Genetics, Vol. 118, pp. 581-592 (1988)]. Using this procedure, the *sly*A gene was mapped to a region between 28.5 and 30 minutes. Few genes have been identified in this location, either in *Salmonella* or in the related region in *E. coli* (F.C. Neidhard, *Escherichia coli and Salmonella typhimurium*, Cellular and Molecular Biology, (American Society of Microboloby, Washington, D.C. (1987)].

DNA was extracted from the parent strain, 17 clinical *Salmonella* isolates, three *Shigella* serotypes, enteroinvasive *E. coli* (EIEC), and other pathogenic bacteria [Materials and Methods for list). Southern hybridization using the 680 bp fragment as a probe showed that the gene is present in all serotypes of *Salmonella* examined. *Shigella* and enteroinvasive *E. coli* (EEEC) (Figure 2) but not in any of the other 25 bacterial species examined (data not shown). A 7 kb fragment hybridized in most serotypes of *Salmonella*. Three serotypes showed two fragments that hybridized, suggesting that the *sly*A gene may be duplicated in some serotypes of *Salmonella* or there are several Pstl sites in or around the *sly*A gene.

The following Table 1 summarizes these results for the genus *Salmonella.*

**TABLE 1:**

| Sample | Fragment | | |
|---|---|---|---|
| | 1.5 Kbp | 5 Kbp | 7 Kbp |
| Salmonella | | | |
| typhimurium 14028a | - | (+) | + |
| typhimurium (clinical) | - | - | + |
| typhimurium (clinical) | - | - | + |
| heidelberg | - | - | + |
| montevideo | - | - | + |
| infantis | - | - | +*) |
| choleraesuis | + | - | + |
| newport | - | - | + |
| hadar | - | + | - |
| enteritidis | - | - | +*) |
| havana | - | - | + |
| barta | - | - | + |
| typhi | - | - | + |
| typhi 21A | - | - | + |
| worthington | + | - | + |
| cerro | - | - | + |
| arizona | - | - | + |
| typhisuis | - | - | + |

| | | | |
|---|---|---|---|
| Explanations: + band with given length of fragment (1.5, 5, 7 Kbp) (+) faint band with given length of fragment (1.5, 5, 7 Kbp) - no band with given length of fragment (1.5, 5, 7 Kbp) *) multiple bands around 7 Kbp | | | |

### EXAMPLE 3: BIOLOGICAL PROPERTIES OF SALMOLYSIN

The size of the *sly* gene product was determined. *Sly*A was over-expressed by placing the coding region under the control of T7 RNA polymerase using the PCR primers shown in Figure 1. A 519 bp fragment was amplified, cloned into pRK172 and transformed into *E. coli* BL21 DE [F.W. Studier et al., Methods in Enzymology, Vol. 185, pp. 6-89 (1990)]. Proteins were labeled in the presence of IPTG (which induces expression of T7 RNA polymerase) and ³⁵S-methionine, then separated by SCS-PAGE. A single 16,000 dalton protein was specifically labeled. These results are in close agreement with the size of salmolysin predicted from the DNA sequence.

Isolation of functional salmolysin was facilitated by the discovery that the hemolytic activity of the *E. coil* clones expressing the salmolysin gene could be released by osmotic shock. Overnight cultures of *E. coli* expressing cloned salmolysin (pSL1117) were osmotically shocked [F.W. Studier et al., Methods in Enzymology, Vol. 165, pp. 6-89 (1990)]. Released salmolysin was partially purified by FPLC MonoQ anion exchange chromatography (Pharmacia); all hemolytic activity eluted at 50 mM NaCl. A 16,000 dalton protein, identical in size to the protein over-expressed under the control of T7 RNA polymerase, was enriched in this fraction. Identically processed osmotic shock supernatants of *E. coli* with pKS alone had no protein in this size range that eluted at 150 mM NaCl, nor was hemolysin activity detected in any fraction. Salmolysin was found to have an isoelectric point of pl 5.5 determined by preparative isoelectric focusing. Some of the biochemical properties of salmolysin were determined. The hemolytic activity of salmolysin was found to be sensitive to heat (65°C for 15 minutes) and was fully active in the presence of 50 mM EDTA, unlike the hemolysin from uropathogenic *E. coli* [ F.C. Neidhard, *Escherichia coli and Salmonella typhimurium*, Cellular and Molecular Biology, (American Society of Microboloby, Washington, D.C. (1987)]. No phospholipase or cholesterol oxidase activity was detected. Salmolysin is also able to lyse nucleated cells as well as a variety of erythrocytes (data not shown). Based on these results, a salmolysin is called a cytolysin.

The preceding examples can be repeated with similar success by substituting the generically or specifically described reactants and/or operating conditions of this invention for those used in the preceding examples.

## Claims

1. Oligonucleotide selected from a DNA sequence according to SEQU.ID.NO.1 which is the nucleic acid sequence of the slyA gene, having a sequence according to SEQU.ID.NO.3, SEQU.ID.NO.4 or SEQU.ID.NO.5.

2. A method of detecting the presence of Salmonella comprising
- hybridizing an oligonucleotide according to claim 1 with a nucleic acid-containing sample suspected to contain Salmonella under conditions that allow selective detection of the slyA gene in that sample and
- determining the presence or absence of binding of the oligonucleotide, the presence of binding being indicative of the presence of Salmonella in the sample wherein said slyA gene is a nucleic acid having the sequence shown in Figure 1 or a sequence hybridizing to the DNA sequence of Figure 1 over its entire length and whereby the encoded protein has the biological activity of the protein encoded by the DNA sequence of Figure 1.

3. The method of claim 2, wherein the sample is a food stuff.

4. The method of claim 2, wherein the sample is blood, serum, plasma, urine or feces.

5. A method of detecting the presence of Salmonella comprising
- hybridizing two different oligonucleotides comprising a DNA sequence which is specific for the DNA sequence of Figure 1, at least one of the oligonucleotides being an oligonucleotide according to claim 1 or their complements with a nucleic acid-containing sample suspected to contain Salmonella under conditions that allow selective detection of the slyA gene in that sample
- performing the polymerase chain reaction on the hybridized sample and
- determining the presence or absence of a DNA fragment formed by the polymerase chain reaction, the presence being indicative of the presence of Salmonella in the sample
wherein said slyA gene is a nucleic acid having the sequence shown in Figure 1 or a sequence hybridizing to the DNA sequence of Figure 1 over its entire length and whereby the encoded protein has the biological activity of the protein encoded by the DNA sequence of Figure 1.

6. A kit for detecting the presence of Salmonella comprising
- a first container having an oligonucleotide comprising a sequence according to claim 1 or a sequence which is complementary to a sequence of claim 1 and
- a second container having one or more components necessary for carrying out the reaction.

7. A kit for amplification of a region of DNA comprising slyA gene comprising
- a pair of single-stranded nucleic acid sense and antisense primers which are specific for the DNA sequence of Figure 1, at least one primer having a sequence or being complementary to a sequence according to claim 1
wherein said slyA gene is a nucleic acid having the sequences shown in Figure 1 or a sequence hybridizing to the DNA sequence of Figure 1 over its entire length and whereby the encoded protein has the biological activity of the protein encoded by the DNA sequence of Figure 1.

## Patentansprüche

1. Oligonukleotid, ausgewählt aus einer DNA-Sequenz gemäß SEQU.ID.NO.1, bei der es sich um die Nukleinsäuresequenz des slyA-Gens handelt, mit einer Sequenz gemäß SEQU.ID.NO.3, SEQU.ID.NO.4 or SEQU.ID.NO.5.

2. Verfahren zum Nachweis der Anwesenheit von Salmonella, bei dem
- ein Oligonukleotid nach Anspruch 1 mit einer nukleinsäurehaltigen Probe, von der man annimmt, daß sie Salmonella enthält, unter Bedingungen, die den selektiven Nachweis des slyA-Gens in der Probe gestatten, hybridisiert und
- das Vorliegen oder Nichtvorliegen der Bindung des Oligonukleotids bestimmt wird, wobei das Vorliegen einer Bindung die Anwesenheit von Salmonella in der Probe anzeigt, wobei es sich bei dem slyA-Gen um eine Nukleinsäure mit der in Figur 1 gezeigten Sequenz oder einer mit der DNA-Sequenz in Figur 1 über ihre gesamte Länge hybridisierende Sequenz handelt und wodurch das codierte Protein die biologische Aktivität des von der DNA-Sequenz in Figur 1 codierten Proteins besitzt.

3. Verfahren nach Anspruch 2, wobei es sich bei der Probe um ein Lebensmittel handelt.

4. Verfahren nach Anspruch 2, wobei es sich bei der Probe um Blut, Serum, Plasma, Urin oder Faeces handelt.

5. Verfahren zum Nachweis der Anwesenheit von Salmonella, bei dem
- zwei verschiedene Oligonukleotide, die eine für die DNA-Sequenz in Figur 1 spezifische DNA-Sequenz umfassen, wobei es sich bei wenigstens einem der Oligonukleotide um ein Oligonukleotid nach Anspruch 1 handelt, oder ihre Komplemente mit einer nukleinsäurehaltigen Probe, von der man annimmt, daß sie Salmonella enthält, unter Bedingungen, die den selektiven Nachweis des slyA-Gens in der Probe gestatten, hybridisiert werden,
- die Polymerasekettenreaktion mit der hybridisierten Probe durchgeführt und
- das Vorliegen oder Nichtvorliegen eines durch die Polymerasekettenreaktion gebildeten DNA-Fragments bestimmt wird, wobei das Vorliegen die Anwesenheit von Salmonella in der Probe anzeigt,
wobei es sich bei dem slyA-Gen um eine Nukleinsäure mit der in Figur 1 gezeigten Sequenz oder einer mit der DNA-Sequenz in Figur 1 über ihre gesamte Länge hybridisierende Sequenz handelt und wodurch das codierte Protein die biologische Aktivität des von der DNA-Sequenz in Figur 1 codierten Proteins besitzt.

6. Kit zum Nachweis der Anwesenheit von Salmonella, umfassend
- einen ersten Behälter mit einem Oligonukleotid, das eine Sequenz nach Anspruch 1 oder eine zu einer Sequenz nach Anspruch 1 komplementäre Sequenz umfaßt, und
- einen zweiten Behälter mit einer oder mehreren zur Durchführung der Reaktion notwendigen Komponenten.

7. Kit zur Amplifikation eines das slyA-Gen umfassenden DNA-Bereichs, umfassend
- ein Paar einzelsträngiger Sense- und Antisense-Nukleinsäureprimer, die spezifisch für die DNA-Sequenz in Figur 1 sind, wobei wenigstens ein Primer eine Sequenz nach Anspruch 1 aufweist oder dazu komplementär ist,
wobei es sich bei dem slyA-Gen um eine Nukleinsäure mit der in Figur 1 gezeigten Sequenz oder einer mit der DNA-Sequenz in Figur 1 über ihre gesamte Länge hybridisierende Sequenz handelt und wodurch das codierte Protein die biologische Aktivität des von der DNA-Sequenz in Figur 1 codierten Proteins besitzt.

## Revendications

1. Oligonucléotide sélectionné à partir d'une séquence d'ADN selon SEQU.ID.NO.1 qui est la séquence d'acide nucléique du gène slyA, ayant une séquence selon SEQU.ID.NO.3, SEQU.ID.NO.4 ou SEQU.ID.NO.5.

2. Méthode de détection de la présence de Salmonella comprenant les étapes consistant :
- à hybrider un oligonucléotide selon la revendication 1 avec un échantillon contenant des acides nucléiques suspecté de contenir Salmonella, dans des conditions qui permettent la détection sélective du gène slyA dans cet échantillon et
- à déterminer la présence ou l'absence de liaison de l'oligonucléotide, la présence d'une liaison indiquant la présence de Salmonella dans l'échantillon, dans laquelle ledit gène slyA est un acide nucléique ayant la séquence présentée à la figure 1 ou une séquence s'hybridant à la séquence d'ADN de la figure 1 sur toute sa longueur et grâce à quoi la protéine codée a l'activité biologique de la protéine codée par la séquence d'ADN de la figure 1.

3. Méthode selon la revendication 2, dans laquelle l'échantillon est un produit alimentaire.

4. Méthode selon la revendication 2, dans laquelle l'échantillon est du sang, du sérum, du plasma, de l'urine ou des fèces.

5. Méthode de détection de la présence de Salmonella comprenant les étapes consistant :
- à hybrider deux oligonucléotides différents comprenant une séquence d'ADN qui est spécifique de la séquence d'ADN de la figure 1, au moins un des oligonucléotides étant un oligonucléotide selon la revendication 1 ou leurs compléments, avec un échantillon contenant des acides nucléiques suspecté de contenir Salmonella, dans des conditions qui permettent la détection sélective du gène slyA dans cet échantillon
- à mettre en oeuvre la réaction de polymérisation en chaîne sur l'échantillon hybridé et
- à déterminer la présence ou l'absence d'un fragment d'ADN formé par la réaction de polymérisation en chaîne, la présence indiquant la présence de Salmonella dans l'échantillon,
dans laquelle ledit gène slyA est un acide nucléique ayant la séquence présentée à la figure 1 ou une séquence s'hybridant à la séquence d'ADN de la figure 1 sur toute sa longueur et grâce à quoi la protéine codée a l'activité biologique de la protéine codée par la séquence d'ADN de la figure 1.

6. Kit pour détecter la présence de Salmonella comprenant
- un premier récipient contenant un oligonucléotide comprenant une séquence selon la revendication 1 ou une séquence qui est complémentaire d'une séquence de la revendication 1 et
- un second récipient contenant un ou plusieurs composants nécessaires pour mettre en oeuvre la réaction.

7. Kit pour amplifier une région d'ADN comprenant le gène slyA, comprenant
- une paire d'amorces d'acide nucléique monocaténaire sens et antisens qui sont spécifiques de la séquence d'ADN de la figure 1, au moins une amorce ayant une séquence ou étant complémentaire d'une séquence selon la revendication 1, dans lequel ledit gène slyA est un acide nucléique ayant les séquences présentées à la figure 1 ou une séquence s'hybridant à la séquence d'ADN de la figure 1 sur toute sa longueur et grâce à quoi la protéine codée a l'activité biologique de la protéine codée par la séquence d'ADN de la figure 1.


### SEQUENCE LISTING

(1) GENERAL INFORMATION:
   (i) APPLICANT:
      (A) NAME: Merck Patent GmbH
      (B) STREET: Frankfurter Str. 250
      (C) CITY: Darmstadt
      (E) COUNTRY: Germany
      (F) POSTAL CODE (ZIP): 64271
      (G) TELEPHONE: 06151-727840
      (H) TELEFAX: 06151-727191
      (I) TELEX: 4 19 328 32 em d
   (ii) TITLE OF INVENTION: Cytolysin Gene and its Use for the Detection of Enterobacteriaceae
   (iii) NUMBER OF SEQUENCES: 5
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: WO
   (vi) PRIOR APPLICATION DATA:
      (A) APPLICATION NUMBER: US 08/054,480
      (B) FILING DATE: 30-APR-1993
   (vi) PRIOR APPLICATION DATA:
      (A) APPLICATION NUMBER: US 08/054,395
      (B) FILING DATE: 28-APR-1993
(2) INFORMATION FOR SEQ ID NO:1:
   (i) SEQUENCE CHARACTERISTICS:
      (A) LENGTH: 720 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Salmonella
   (xi) SEQUENCE DESCRIPTION: SEQ ID NO:1:
   ATCGATGCTT TAGTTTTAGC CAAAACTGAA GCTACAGGTG CCAAGTGCGC ACTATGTCTG    60
   AAAAAATGTC TATTGGTAAG CAAATTTAGC AATACATTTG TTTTGAGAAT ACAAATACTG   120
   CACACTATTC TAAAATCAGC ATAATAACTT AGCAAGCTAA TTATAAGGAC ATGAAATTGG   180
   AATCGCCACT AGGTTCTGAT CTGGCACGGT TGGTGCGCAT TTGGCGTGCT CTGATTGACC   240
   ATCGCCTCAA GCCTCTGGAA TTGACGCAGA CACATTGGGT CACGTTGCAC AATATTCATC   300
   AATTGCCGCC TGACCAGTCG CAGATTCAAT TGGCTAAAGC GATAGGCATT GAGCAGCCAT   360
   CGCTGGTACG CACGTTGGAT CAACTTGAAG ATAAGGGGCT AATTTCGCGG CAAACCTGCG   420
   CCAGCGATCG TCGCGCTAAG CGGATTAAAC TGACCGAAAA AGCGGAGCCG CTGATCGCTG   480
   AGATGGAAGA GGTCATTCAT AAAACGCGCG GTGAAATTTT GGCTGGGATT TCTTCAGAGG   540
   AGATTGAGCT TCTGATTAAA CTTATCGCCA AACTTGAACA CAATATTATG GAATTGCACT   600
   CTCACGATTG AGGTGCAGGG GCATACGTGT GGCCATGTGA CCACACGTAA AGCCTGGTTT   660
   AGCGTGGAGA GACGGTAACC TGGCTGCCGT TGCTGGCCAG CACGACACGC TGACCTGCCG   720
(2) INFORMATION FOR SEQ ID NO:2:
   (i) SEQUENCE CHARACTERISTICS:
      (A) LENGTH: 146 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Salmonella
   (xi) SEQUENCE DESCRIPTION: SEQ ID NO:2:
   Met Lys Leu Glu Ser Pro Leu Gly Ser Asp Leu Ala Arg Leu Val Arg
   1 5 10 15
   Ile Trp Arg Ala Leu Ile Asp His Arg Leu Lys Pro Leu Glu Leu Thr
   20 25 30
   Gln Thr His Trp Val Thr Leu His Asn Ile His Gln Leu Pro Pro Asp
   35 40 45
   Gln Ser Gln Ile Gln Leu Ala Lys Ala Ile Gly Ile Glu Gln Pro Ser
   50 55 60
   Leu Val Arg Thr Leu Asp Gln Leu Glu Asp Lys Gly Leu Ile Ser Arg
   65 70 75 80
   Gln Thr Cys Ala Ser Asp Arg Arg Ala Lys Arg Ile Lys Leu Thr Glu
   85 90 95
   Lys Ala Glu Pro Leu Ile Ala Glu Met Glu Glu Val Ile His Lys Thr
   100 105 110
   Arg Gly Phe Ile Leu Ala Gly Ile Ser Ser Glu Glu Ile Phe Leu Leu
   115 120 125
   Ile Lys Leu Ile Ala Lys Leu Glu His Asn Ile Met Glu Leu His Ser
   130 135 140
   His Asp
   145
(2) INFORMATION FOR SEQ ID NO:3:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Salmonella
   (xi) SEQUENCE DESCRIPTION: SEQ ID NO:3:
   ATCGATGCTT TAGTTTTAGC CAA 23
(2) INFORMATION FOR SEQ ID NO:4:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Salmonella
   (xi) SEQUENCE DESCRIPTION: SEQ ID NO:4:
   ATTATAAGGA GATGAAATTG GAATCGCC 28
(2) INFORMATION FOR SEQ ID NO:5;
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Salmonella
   (xi) SEQUENCE DESCRIPTION: SEQ ID NO:5:
   TTAGCGTGGA GAGACGGTAA CCTGGC 26